**Europäisches Patentamt**

(19) **European Patent Office**

**Office européen des brevets**

(11) Numéro de publication: **0 114 780**
**B1**

(12) **FASCICULE DE BREVET EUROPEEN**

(45) Date de publication du fascicule du brevet:
**17.11.88**

(21) Numéro de dépôt: **84400147.9**

(22) Date de dépôt: **24.01.84**

(51) Int. Cl.⁴: **G 01 N 33/18**

(54) Procédé et appareil de détection de substances toxiques dans l'eau résiduaire alimentant une station de traitement biologique.

(30) Priorité: **24.01.83 FR 8301027**

(43) Date de publication de la demande:
**01.08.84 Bulletin 84/31**

(45) Mention de la délivrance du brevet:
**17.11.88 Bulletin 88/46**

(84) Etats contractants désignés:
**AT BE CH DE GB IT LI LU NL**

(56) Documents cités:
**EP - A - 0 009 580**
**FR - A - 2 067 184**
**FR - A - 2 428 842**
**US - A - 4 162 195**

(73) Titulaire: **ATOCHEM, 4 & 8, Cours Michelet La Défense 10, F-92800 Puteaux (FR)**

(72) Inventeur: **Copiot, Daniel, 1, Allée des Daphnés Le Plessis Bouchard, F-95130 Franconville (FR)**
Inventeur: **Picon, Georges, 54, blvd J.-J. Rousseau, F-92230 Gennevilliers (FR)**

(74) Mandataire: **Joly, Jean-Jacques et al, CABINET BEAU DE LOMENIE 55, rue d'Amsterdam, F-75008 Paris (FR)**

## Description

La présente invention concerne un procédé de détection de substances toxiques dans l'eau résiduaire alimentant une station de traitement biologique.

Dans les stations de traitement biologique des eaux résiduaires, les effluents sont mis en contact avec des micro-organismes qui métabolisent les matières organiques dissoutes en présence d'oxygène.

L'activité de ces micro-organismes peut être inhibée partiellement ou totalement lorsque des matières toxiques sont présentes dans les eaux résiduaires, à une concentration telle que se manifeste un empoisonnement de la biomasse épuratrice, se traduisant par une diminution ou un arrêt de l'épuration.

Lorsque la présence de toxiques n'est pas décelée suffisamment tôt, toute la biomasse de la station peut être empoisonnée et il faut alors plusieurs semaines pour que l'installation d'épuration retrouve son efficacité. L'intérêt de déceler la présence de substances toxiques dans les eaux résiduaires, avant que leur effet ne se manifeste au niveau de la station d'épuration, est donc évident.

De nombreux procédés de détection de substances toxiques sont déjà connus et exploités, notamment des procédés basés sur des mesures respirométriques: les toxiques agissent en modifiant le métabolisme des micro-organismes avec lesquels ils sont mis en présence, et on peut détecter avec beaucoup de sensibilité une variation d'activité métabolique en suivant une fonction essentielle: la respiration.

Ainsi, selon le procédé décrit dans la demande de brevet français FR 2 428 842, le liquide à tester est saturé en oygène et introduit dans un réacteur où il est mélangé à une fraction de culture de bactéries et à un nutrient. La culture est préparée de manière à avoir une suspension aqueuse bactérienne à caractéristiques stables. Le liquide est introduit à l'entrée du réacteur formé par un tube long et mince et la teneur en oxygène est mesurée à la sortie du réacteur, à l'autre extrémité du tube; les pics détectés sur l'enregistrement de la valeur mesurée de teneur en oxygène traduisent la présence de toxiques. Un procédé similaire est décrit dans la demande de brevet FR 2 266 885 puisqu'il consiste à saturer en oxygène une partie de l'effluent à surveiller et à la faire passer à travers un filtre bactérien en sortie duquel l'oxygène dissous est mesuré. Une consommation d'oxygène nulle ou trop faible déclenche l'alarme de toxicité. Le renouvellement de l'ensemencement du réacteur est réalisé de façon périodique et une dilution de l'effluent par une solution nutritive s'avère nécessaire lorsque les variations de charge de l'effluent sont trop importantes. Avec des procédés de ce type, les micro-organismes utilisés dans le réacteur peuvent réagir différemment de ceux présents dans la station d'épuration. De plus, la dilution de l'effluent par une solution nutritive produit une dilution du toxique qui retarde sa détection.

Le brevet U.S. 4 260 490 décrit quant à lui un procédé selon lequel la respiration mesurée sur un échantillon de boues activées de la station est comparée à la respiration mesurée sur un échantillon de boues activées de la station auquel est ajouté l'effluent à surveiller. Si la différence entre les vitesses de consommation d'oxygène est inférieure à une valeur déterminée avec un effluent dépourvu de toxicité et servant de référence, l'effluent surveillé est toxique. Si, au contraire, cette différence est supérieure à celle déterminée avec un effluent de demande biologique en oxygène moyenne, la demande en oxygène est plus importante. Un calculateur interprète les résultats des tests respirométriques et commande, le cas échéant, une alarme de toxicité ou de surcharge de la station. Il s'agit donc d'un procédé discontinu nécessitant, en plus d'un calculateur, deux sondes à oxygène ainsi que deux analyseurs.

Suivant encore un autre procédé, du type de ceux décrits dans les brevets FR 1 567 181, FR 1 567 182 et FR 2 067 184, on utilise un réacteur recevant une certaine quantité de boues activées en provenance de la station et oxygénées par injection d'air comprimé. Lorsque ces boues ont atteint une respiration constante, un échantillon d'effluent à contrôler est injecté. La quantité d'oxygène absorbé dès cet instant est mesurée en continu et un calculateur fournit la courbe de métabolisation. Ensuite, si au cours de mesures ultérieures, la respiration devient inférieure à la valeur déterminée initialement, la probabilité de présence de toxiques est très élevée. Il s'agit là d'un procédé de surveillance discontinue.

Enfin, il est décrit dans la demande de brevet européen EP 0 009 580 un système permettant la commande d'une installation d'épuration biologique au moyen d'un dispositif de mesure pouvant recevoir un mélange d'eau résiduaire et de boues recyclées prélevées avant leur entrée dans le bassin de l'installation. Le dispositif de mesure comprend un réacteur, dans lequel la suspension arrive et est saturée en oxygène, et une cellule de mesure espacée du réacteur et destinée à la mesure de la consommation en oxygène. Le trajet entre le réacteur et la cellule de mesure est défini de manière à correspondre à une durée réactionnelle déterminée préalablement. Si la valeur mesurée de consommation en oxygène franchit des premiers seuils maximal et minimal prédéterminés, on agit sur le débit de boues recyclées vers la station d'épuration. Le franchissement d'un second seuil minimal inférieur au premier est considéré comme significatif d'un empoisonnement.

La présente invention a pour but de fournir un procédé et un dispositif destinés spécifiquement à la surveillance en continu de la toxicité des eaux résiduaires, ceci dans les conditions les plus proches possible de celles d'exploitation de la station d'épuration et en donnant une réponse très rapide par suite de l'apparition de toxiques.

A cet effet, l'invention se rapporte à un procédé du type selon lequel de l'eau résiduaire à surveiller est mise en contact dans un réacteur avec des boues activées provenant de la station de traitement biologique, un apport constant en oxygène est réalisé dans le réacteur, et la teneur en oxygène dissous dans le réacteur est mesurée afin de déclencher une alarme de toxicité en cas de diminution anormale de la respiration, procédé selon lequel, conformément à l'invention, le réacteur est alimenté de façon continue par des boues activées provenant de la station; la teneur mesurée en oxygène dissous dans le réacteur est comparée en permanence à une valeur minimale et à une valeur maximale prédéterminées; le débit d'alimentation du réacteur en eau résiduaire est automatiquement régulé en étant diminué ou augmenté lorsque la teneur mesurée devient, respectivement, inférieure à la valeur minimale ou supérieure à la valeur maximale, de manière à maintenir sensiblement constante la charge appliquée au réacteur; et l'alarme de toxicité est déclenchée lorsque la teneur mesurée reste supérieure à ladite valeur maximale alors que le débit d'alimentation du réacteur en eau résiduaire est à un maximum.

L'alimentation du réacteur en permanence par des boues provenant de la station de traitement et par l'eau résiduaire destinée à cette station permet de s'approcher au plus près des conditions réelles d'exploitation de la station. En outre, par la régulation automatique du débit d'admission d'eau résiduaire, la charge polluante appliquée est maintenue sensiblement constante et une diminution anormale de la respiration peut être imputée avec certitude à la présence de substance toxique dans l'eau résiduaire. L'alarme est déclenchée en cas d'impossibilité de stabiliser la teneur en oxygène dissous à un niveau donné en agissant sur la charge du réacteur; ainsi, le fait d'augmenter le débit d'eau résiduaire en cas d'augmentation de la teneur en oxygène dissous permet d'accélérer la détection de la toxicité.

La présente invention a aussi pour but de fournir un appareil permettant la mise en œuvre du procédé.

Ce but est atteint grâce à un appareil du type comportant un réacteur destiné à contenir des boues activées provenant de la station de traitement biologique, un dispositif d'alimentation du réacteur en oxygène, un dispositif d'alimentation du réacteur en eau résiduaire à surveiller, un dispositif de mesure de la teneur en oxygène dissous dans le réacteur et un dispositif d'alarme relié au dispositif de mesure pour déclencher une alarme de toxicité en cas de diminution anormale de la respiration, appareil dans lequel, conformément à l'invention, sont prévus: un dispositif pour alimenter en permanence le réacteur par des boues activées en provenance de la station et un dispositif de régulation qui comporte un circuit comparateur destiné à comparer la valeur mesurée de la teneur en oxygène dissous à une valeur minimale et à une valeur maximale prédéterminées, un circuit de commande d'une pompe d'alimentation du réacteur en eau résiduaire, et un circuit de sélection relié au circuit comparateur et destiné à appliquer au circuit de commande un signal différent selon que la teneur en oxygène mesurée est inférieure à ladite valeur minimale, supérieure à ladite valeur maximale ou comprise entre les deux, de façon à diminuer au augmenter le débit d'alimentation du réacteur en eau résiduaire lorsque la teneur mesurée devient, respectivement, inférieure à la valeur minimale ou supérieure à la valeur maximale.

D'autres particularités et avantages du procédé et de l'appareil conformes à l'invention ressortiront à la lecture de la description faite ci-après, à titre indicatif mais non limitatif, en référence aux dessins annexés sur lesquels:

– la figure 1 est une vue très schématique d'un mode de réalisation d'un appareil conforme à l'invention,

– la figure 2 est un schéma des circuits du dispositif de régulation de débit d'eau résiduaire et du dispositif d'alarme de l'appareil de la figure 1, et

– les figures 3 à 6 sont des courbes illustrant les variations de la teneur en oxygène dissous dans le réacteur d'un appareil conforme à l'invention, en réponse à des variations de charge ou à l'apparition de toxiques dans l'eau résiduaire surveillée.

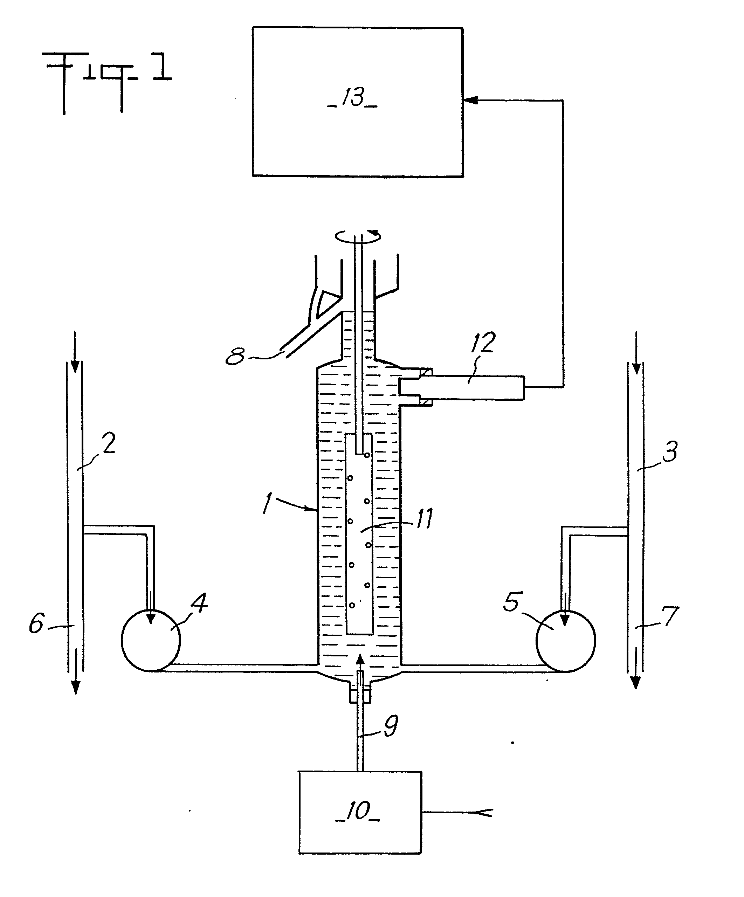

L'appareil représenté schématiquement sur la figure 1 comprend un réacteur 1 alimenté en eau résiduaire dans laquelle la présence de substances toxiques est à détecter, et en boues activées provenant d'une station de traitement biologique (non représentée) destinée à l'épuration de l'eau résiduaire surveillée.

Les boues biologiques peuvent être prélevées dans les bassins de boues activées ou, de préférence dans le circuit de recyclage des boues après concentration de celles-ci dans le décanteur de la station. L'eau résiduaire à surveiller est prélevée en amont de la station ou du bassin d'homogénéisation lorsqu'il y en a un. L'appareil de détection des toxiques est installé de préférence le plus près possible des prélèvements de boues et d'eau résiduaire de façon que le temps écoulé entre la prise de boues, d'eau résiduaire et leur arrivée à l'appareil soit seulement de quelques minutes. Pour que ce temps soit très court et pour éviter un dépôt dans les canalisations d'amenée d'eau résiduaire et de boues, on réalise des boucles d'alimentations rapides respectives 2 et 3. On peut utiliser la pression hydrostatique ou la pression de refoulement de pompes déjà installées sur la station: pompe de recyclage des boues, par exemple. Des piquages sur ces boucles rapides, au niveau de l'appareil, permettent l'alimentation du réacteur 1, en eau résiduaire par la pompe péristaltique 4, en boues par la pompe péristaltique 5. L'excédent d'eau résiduaire, de boues et la surverse du réacteur 1 sont recyclés en tête de station par des canalisations respectives 6, 7 et 8. L'alimentation du réacteur 1 en boues activées et en eau résiduaire à surveiller est réalisée de façon permanente.

En outre, un apport constant d'oxygène dans le réacteur 1 est réalisé par barbotage d'air comprimé au moyen d'un tube poreux ou effilé 9 qui traverse le fond du réacteur. La pression et le débit de l'air comprimé amené au tube 9 sont régulés et contrôlés par un dispositif de régulation 10. La dissolution de l'oxygène est facilitée par un agitateur 11 tournant à une vitesse aussi constante que possible.

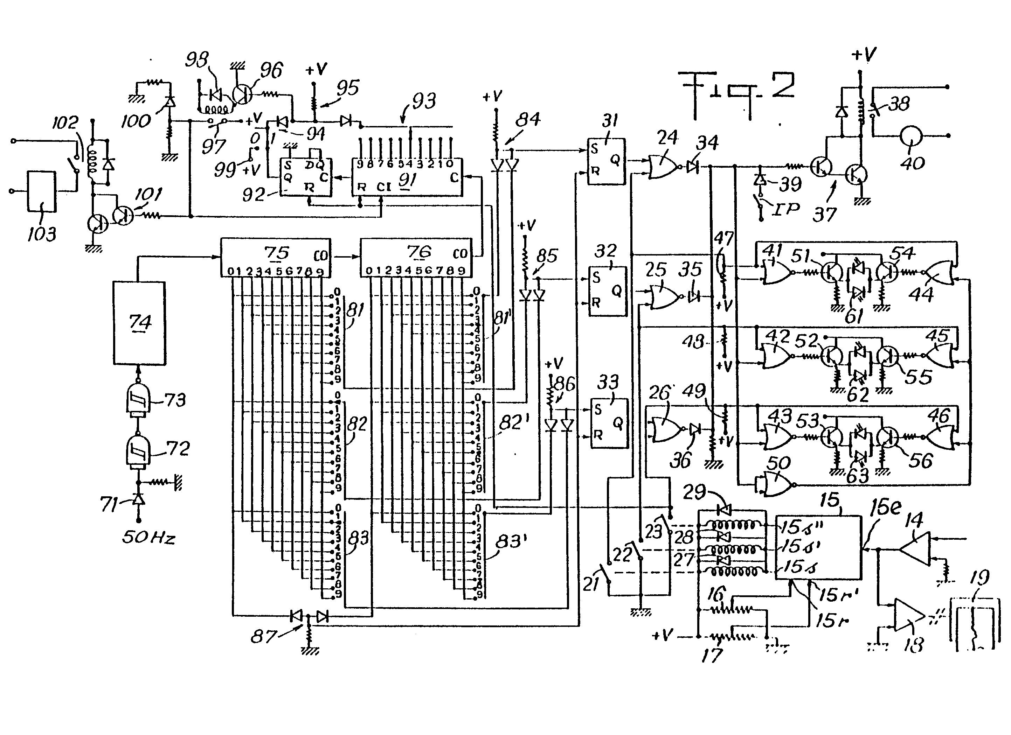

Une sonde à oxygène 12 de type polarographique fournit un signal représentatif de la teneur en oxygène dissous dans le réacteur. La sonde 12 est disposée au voisinage de la sortie du réacteur 1, à la partie supérieure de celui-ci. Le signal de la sonde 12 est appliqué à un dispositif de régulation et d'alarme 13 qui est décrit ci-après en détail en référence à la figure 2.

La variation de la concentration en oxygène dissous dans le réacteur permet de déterminer si la charge polluante appliquée est normale, forte, faible ou présente une toxicité, selon que cette concentration en oxygène dissous se maintiendra, diminuera ou augmentera.

Selon une caractéristique de la présente invention, le débit d'alimentation du réacteur 1 en eau résiduaire est commandé en fonction de la valeur mesurée de la concentration en oxygène dissous. Si la concentration diminue et devient inférieure à une valeur de seuil minimal prédéterminée, signe d'une augmentation de la charge appliquée, le débit d'eau résiduaire est diminué. Par contre, si la concentration augmente et devient supérieure à une valeur de seuil maximal prédéterminée, signe d'une diminution de la charge appliquée ou de la présence de toxiques, le débit d'eau résiduaire est augmenté; s'il s'agissait d'une diminution de la charge, la concentration en oxygène dissous doit retrouver un niveau normal; s'il s'agissait d'une apparition de toxiques, la concentration en oxygène croît encore de façon anormale ce qui déclenche l'alarme: l'augmentation du débit d'eau résiduaire accélère le processus de détection, ce qui est un des avantages apportés par l'invention.

La variation du débit d'eau résiduaire en vue de réguler la charge appliquée au réacteur peut être réalisée de façon continue ou par paliers. Dans ce dernier cas, plusieurs valeurs de débit d'eau résiduaire peuvent être prédéterminées, le passage d'une valeur à une autre étant commandé en réponse à un franchissement du seuil minimal ou maximal de la concentration en oxygène dissous.

La comparaison entre la valeur mesurée de la concentration et les seuils prédéterminés est assurée au sein du dispositif de régulation et d'alarme 13 qui délivre des signaux destinés notamment à commander la pompe 4 pour fixer le débit d'eau résiduaire amenée au réacteur et à déclencher, le cas échéant, une alarme de toxicité.

Ce signal produit par la sonde 12 est amplifié au moyen d'un circuit amplificateur 14 à gain réglable. Après amplification, le signal est appliqué à l'entrée 15e d'un discriminateur à fenêtre 15. Ce circuit comporte trois sorties 15s, 15s', 15s'' qui sont activées lorsque l'amplitude du signal à l'entrée 15e est respectivement comprise dans une plage ou fenêtre prédéterminée [C − ΔC, C

+ ΔC], inférieure à C−ΔC ou supérieure à C + ΔC. La valeur C du milieu de fenêtre est ajustable au moyen d'un potentiomètre 16 formant diviseur de tension entre la masse et une borne au potentiel +V de la tension d'alimentation et appliquant une tension ajustable sur une première entrée de réglage 15r du discriminateur 15. De même, la largeur 2 ΔC de la fenêtre est ajustable au moyen d'un autre potentiomètre 17 formant diviseur de tension et appliquant une tension ajustable sur une seconde entrée de réglage 15r' du discriminateur. Ce dernier est un circuit connu en soi, disponible dans le commerce sous forme de circuit intégré, par exemple le circuit référencé TCA 965 fabriqué par la Société SIEMENS.

Le signal appliqué à l'entrée du discriminateur 15, après une nouvelle amplification au moyen d'un circuit amplificateur 18, est transmis à un enregistreur 19.

Les sorties 15s, 15s', 15s'' du discriminateur sont respectivement reliées aux bobines d'excitation de trois relais 21, 22, 23 de type Reed dont les contacts mobiles forment interrupteurs entre la masse et des premières entrées respectives de portes 24, 25, 26 de type NON-OU. Des diodes 27, 28, 29 en parallèle sur les bobines des relais 21, 22, 23 protègent les sorties 15s, 15s', 15s''.

Les secondes entrées des portes 24, 25, 26 reçoivent les signaux apparaissant respectivement sur les sorties Q de bascules 31, 32, 33 de type RS. Ces signaux sont sous forme d'impulsions de même fréquence mais de durées différentes. Ainsi, la durée D2 des impulsions en sortie de la bascule 32 est supérieure à celle D1 des impulsions en sortie de la bascule 31 mais inférieure à celle D3 des impulsions en sortie de la bascule 33, ces durées ayant des valeurs prédéterminées réglables, comme décrit plus loin.

Les sorties des portes 24, 25, 26 sont réunies par des diodes 34, 35, 36 à l'entrée d'un circuit amplificateur 37 qui attaque la bobine d'excitation d'un relais 38. Ce dernier, lorsqu'il est fermé, permet l'alimentation du moteur 40 de la pompe 4. Un interrupteur IP actionnable manuellement est branché en série avec une diode 39 entre une borne portée au potentiel +V et l'entrée de l'amplificateur 37, afin de permettre une commande manuelle de la pompe 4.

Selon que le relais 21, 22 ou 23 est fermé, la pompe 4 est mise en marche à intervalles réguliers pour une durée respective D1, D2 ou D3 telles que D1 < D2 < D3. Sont ainsi définis des débits respectivement «bas», «normaux» et «hauts» d'alimentation du réacteur 1 en eau résiduaire.

Les contacts mobiles des relais 21, 22, 23 forment aussi interrupteurs branchés entre la masse et les premières entrées de portes respectives 41, 42, 43 de type NON-OU. Ces premières entrées sont reliées d'une part, aux premières entrées de portes respectives 44, 45, 46 également de type NON-OU et, d'autre part, à une borne au potentiel +V par l'intermédiaire de résistances respectives 47, 48, 49. Les secondes entrées des portes 41, 42, 43 reçoivent le signal appliqué à l'entrée de l'amplificateur 37 et les secondes entrées des portes 44, 45, 46 reçoivent ce signal inversé par une porte 50.

Les sorties des portes 41 à 46 sont reliées par des résistances aux bases de transistors respectifs 51 à 56. Chacun de ces transistors a son collecteur relié à une borne d'alimentation et son émetteur relié à la masse par une résistance. Une double diode électroluminescente 61 est branchée entre les émetteurs des transistors 51 à 54 de façon telle qu'une luminescence rouge soit émise lorsque le transistor 54 est passant et le transistor 51 bloqué. Inversement, une luminescence verte apparaîtra lorsque le transistor 51 sera passant et le transistor 54 bloqué. La diode n'émettra pas quand les deux transistors 51 et 54 seront dans le même état.

De même, une double diode électroluminescente 62 est branchée entre les émetteurs des transistors 52 et 55 et une autre double diode électroluminescente 63 est branchée entre les émetteurs des transistors 53 et 56.

Selon que le relais 21, 22 ou 23 est fermé, les portes 41, 44 ou 42, 45 ou 43, 46 sont validées.

Le signal de commande présent à l'entrée de l'amplificateur 37 commande l'allumage des diodes 61, 62 ou 63 en rouge, tandis que l'absence de ce signal commande l'allumage de ces mêmes diodes en vert. Ainsi, selon que la diode allumée est la 61, 62 ou 63, le débit d'alimentation actuel du réacteur 1 en eau résiduaire est «bas», «normal» ou «haut». L'allumage de cette diode en rouge indique que le moteur de la pompe 4 est alimenté tandis que son allumage en vert indique que le moteur de la pompe 4 n'est pas alimenté.

Les signaux appliqués aux entrées R et S des bascules 31, 32, 33 sont élaborés de la façon suivante.

Un signal à la fréquence du secteur (50 Hz) est prélevé par exemple sur le transformateur d'alimentation puis envoyé à travers une diode 71 à un circuit de mise en forme constitué de deux portes NON-ET à hystérésis 72–73 connectées en série. Le signal carré à 50 Hz en sortie de la porte 73 est appliqué à un diviseur de fréquence 74 qui produit un signal de période égale par exemple à 1/100 de minute. Ce signal est appliqué à l'entrée de comptage d'un compteur-décodeur décimal 75 dont la sortie de retenue CO est reliée à l'entrée de comptage d'un second compteur-décodeur décimal 76. Trois roues codeuses 81, 82, 83 permettent chacune de sélectionner une quelconque des sorties 0 à 9 du compteur 75. De même, trois roues codeuses 81', 82', 83' permettent chacune de sélectionner une quelconque des sorties 0 à 9 du compteur 76.

Les sorties sélectionnées par les roues codeuses 81 et 81' sont réunies par une porte 84 de type ET à diodes dont la sortie est reliée à l'entrée S de la bascule 31. De même, les sorties sélectionnées par les roues codeuses 82 et 82' sont réunies par une porte ET 85 dont la sortie est reliée à l'entrée S de la bascule 32 et les sorties sélectionnées par les roues codeuses 83 et 83' sont réunies par une porte ET 86 dont la sortie est reliée à l'entrée S de la bascule 33. Une autre porte ET 87 a ses entrées reliées aux sorties O des compteurs 75, 76 et a sa sortie reliée aux entrées R des bascules 31, 32 et 33.

Ainsi, les impulsions produites par les bascules 31, 32 et 33 ont, dans l'exemple illustré, une période de 1 mn et des durées D1, D2, D3 exprimées en 1/100e de minute et sélectionnées par les paires de roues codeuses 81–81', 82–82' et 83–83'. On réalise ainsi, pour la commande du moteur de pompe à eau résiduaire, un cycle de 1 mn avec rapport cyclique marche-arrêt variable de 0 à 99% du temps.

Le signal de période 1 mn apparaissant sur la sortie de retenue du compteur 76 est appliqué à l'entrée de comptage d'un compteur-décodeur décimal 91 dont la sortie de retenue est reliée à un autre compteur-décodeur formé par exemple simplement par une bascule 92 de type D. Les entrées R de remise à zéro du compteur 91 et de la bascule 92 sont reliées à la masse par l'intermédiaire de l'interrupteur formé par le contact mobile du relais 23. Une roue codeuse 93 permet de sélectionner l'une quelconque des sorties 0 à 9 du compteur 91 et une seconde roue codeuse 94 permet de sélectionner la valeur 1 ou 0 par connexion à la sortie Q de la bascule 92 ou à une borne 99 portée au potentiel +V. Les bornes ainsi sélectionnées sont reliées aux entrées d'une porte 95 de type ET à diodes dont la sortie est reliée à la base d'un transistor 96. Ce dernier a son émetteur relié à la masse et son collecteur relié à une borne au potentiel +V par l'intermédiaire de la bobine d'excitation d'un relais 97 de type Reed. Une diode de protection 98 est branchée en parallèle sur cette bobine.

Le contact mobile du relais 97 forme interrupteur branché entre une borne portée au potentiel +V et, respectivement, l'entrée CI d'inhibition de comptage du compteur 75, une diode électroluminescente 100 et l'entrée d'un circuit amplificateur 101. Ce dernier attaque la bobine d'excitation d'un relais 102 dont la fermeture commande le fonctionnement d'un circuit d'alarme de toxicité 103.

Lorsque le relais 23 se ferme, les entrées R du compteur 91 et de la bascule 92 passent à zéro et les signaux de sortie du compteur 76 sont pris en compte par le compteur 91. Si le relais 23 reste fermé pendant une durée au moins égale à celle affichée en minutes par les roues codeuses 94 et 93, l'alarme de toxicité est déclenchée et la diode 100 s'allume. L'inhibition du compteur 92 fait que l'alarme est maintenue tant que ce compteur n'est pas remis à zéro. Par contre, si la concentration mesurée redevient normale avant la fin de la temporisation, le relais 23 s'ouvre. Le compteur 91 est remis à zéro ainsi que la bascule 92 et les signaux de sortie du compteur 76 ne sont plus comptés.

Le fonctionnement des circuits décrits ci-avant en référence à la figure 2 découle à l'évidence de ce qui précède.

La valeur de consigne C de la concentration en oxygène dissous dans le réacteur et l'écart admissible ΔC de part et d'autre de cette valeur de consigne sont réglés par l'opérateur au moyen des potentiomètres 16 et 17. Au moyen des roues codeuses 81–81', 82–82' et 83–83', l'opérateur choisit également les valeurs durées de fonctionnement de la pompe 4 au cours de chaque cycle marche-arrêt de celle-ci, c'est-à-dire les valeurs de débit «bas», débit «normal» et débit «haut» d'alimentation du réacteur en eau résiduaire. Ces réglages effectués, le réacteur est rempli en agissant sur l'interrupteur IP de commande manuelle de la pompe 4. Ensuite, le débit de la pompe 4 est commandé automatiquement par le signal de sortie du discriminateur 15, ce qui permet de réaliser une régulation de la charge appliquée au réacteur. Les diodes 61 à 66 permettent à l'opérateur de savoir si le débit d'eau est «bas», «normal» ou «haut», signifiant que la charge est élevée, normale ou faible. Il est ainsi possible, sur la base de cette information, d'agir par exemple sur le débit d'oxygène amené à la station de traitement.

L'opérateur prédétermine également au moyen des roues codeuses 93 et 94 le temps maximum au bout duquel la concentration en oxygène dissous, après avoir dépassé la valeur maximale C + $\Delta$C et commandé la commutation du débit de la pompe 4 à sa valeur «haute», doit revenir dans les limites tolérées, faute de quoi le maintien de la concentration mesurée à un niveau supérieur à C + $\Delta$C est imputé à l'apparition de toxiques. Dans l'exemple illustré, ce temps peut être choisi à une valeur comprise entre 0 et 19 minutes. Grâce au procédé conforme à l'invention, la détection de toxiques est réalisée dans un délais très court en raison de l'effet d'accélération dû au passage sur débit «haut». Grâce à cette détection rapide, les mesures nécessaires peuvent être prises en temps utile pour empêcher une dégradation de la biomasse dans les bassins de la station de traitement.

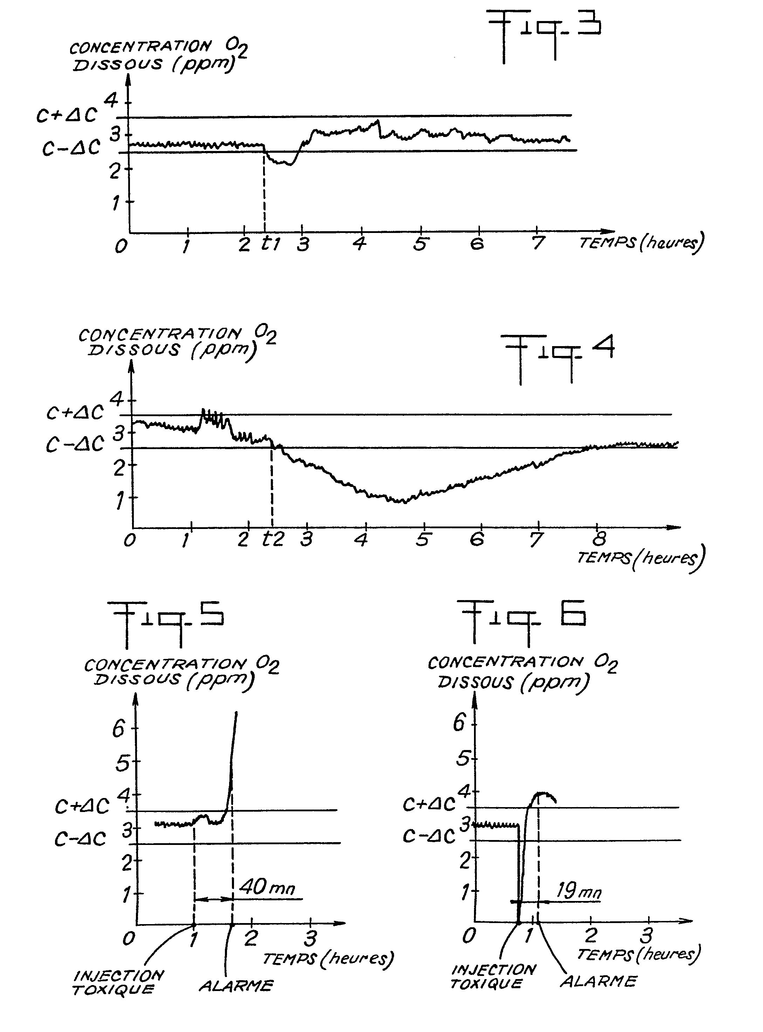

Les figures 3 à 6 illustrent en fonction du temps les variations mesurées de la concentration en oxygène dissous au cours de différents essais de l'apparail décrit ci-avant.

La valeur de consigne de concentration en oxygène dissous a été choisie égale à 3 ppm avec un écart admissible de ±0,5 ppm. Le réacteur, d'une capacité de 2,4 l était alimenté en permanence par des boues prélevées à l'arrivée de l'épaississeur de la station avec un débit de 5,7 l/h et était aéré par un apport constant d'air égal environ à 1 l/h. Les valeurs de débit «bas», «normal» et «haut» ont été choisies égales à 110 ml/h, 220 ml/h et 440 ml/h. La temporisation pour l'alarme de toxicité était réglée à 10 minutes.

La figure 3 montre qu'une augmentation de la charge polluante à l'instant $t_1$ s'est traduite par une diminution de la concentration mesurée, diminution provisoire de durée relativement courte grâce à la compensation par action sur le débit d'eau résiduaire dès le franchissement vers le bas du seuil de 2,5 ppm.

La figure 4 montre qu'une très forte augmentation de charge à l'instant $t_2$ a été compensée dans un délai plus long.

Les figures 5 et 6 représentent les courbes de variation de concentration dans le cas d'introduction volontaire de toxiques, respectivement de l'hypochlorite de sodium à une concentration de 2,3 g de chlore actif par litre d'eau résiduaire et du tertiobutylcatéchol à raison d'1 g introduit dans le réacteur. Dans le premier cas, l'alarme a eu lieu 40 minutes après le début d'introduction. Dans le deuxième cas, il y a d'abord consommation très rapide d'oxygène par suite, vraisemblablement de l'oxydation du tertiobutylcatéchol en quinones très toxiques; ensuite, la concentration en oxygène dissous croît très rapidement provoquant une alarme 20 minutes après l'introduction du tertiobutylcatéchol dans le réacteur.

Suivant le mode de réalisation décrit ci-avant de l'appareil conforme à l'invention, trois niveaux seulement de débit d'eau résiduaire sont prévus. Bien entendu, ce nombre de niveaux peut être supérieur, notamment si d'importantes variations de charge doivent être compensées. On pourra par exemple prévoir un débit «très bas» et un débit «très haut». Par ailleurs, une régulation encore plus fine de la charge peut être réalisée en faisant varier le débit d'eau résiduaire, entre un minimum et un maximum, par incréments très faibles, vorie de façon continue, en fonction de la concentration mesurée en oxygène dissous. Dans tous les cas, l'alarme de toxicité est déclenchée en cas d'impossibilité de maintenir la teneur en oxygène dissous inférieure ou égale à une valeur maximale prédéterminée lorsque le débit d'eau résiduaire est maximum.

## Revendications

1. Procédé de détection de substances toxiques dans l'eau résiduaire alimentant une station de traitement biologique, procédé selon lequel de l'eau résiduaire à surveiller est mise en contact dans un réacteur avec des boues activées provenant de la station de traitement biologique, un apport constant en oxygène est réalisé dans le réacteur, et la teneur en oxygène dissous dans le réacteur est mesurée afin de déclencher une alarme de toxicité en cas de diminution anormale de la respiration,

caractérisé en ce que: le réacteur est alimenté en permanence par lesdites boues activées, la teneur mesurée en oxygène dissous dans le réacteur est comparée en permanence à une valeur minimale et à une valeur maximale prédéterminées; le débit d'alimentation du réacteur en eau résiduaire est automatiquement régulé en étant diminué ou augmenté lorsque la teneur mesurée devient, respectivement, inférieure à la valeur minimale ou supérieure à la valeur maximale, de manière à maintenir sensiblement constante la charge appliquée au réacteur; et l'alarme de toxicité est déclenchée lorsque la teneur mesurée reste supérieure à ladite valeur maximale alors que le débit d'alimentation du réacteur en eau résiduaire est à un maximum.

2. Procédé selon la revendication 1, caractérisé en ce que l'alarme de toxicité est déclenchée lorsque la teneur mesurée en oxygène dissous reste supérieure à une durée prédéterminée comptée à partir du moment où le débit d'alimentation du réacteur en eau résiduaire a atteint son maximum.

3. Procédé selon l'une quelconque des revendications 1 et 2, caractérisé en ce que le débit d'alimentation du réacteur en eau résiduaire est variable par paliers prédéterminés entre un minimum et un maximum.

4. Procédé selon l'une quelconque des revendications 1 et 2, caractérisé en ce que le débit d'alimentation du réacteur en eau résiduaire est variable de façon continue.

5. Appareil de détection de substances toxiques dans l'eau résiduaire alimentant une station de traitement biologique, comportant un réacteur (1) destiné à contenir des boues activées provenant de la station de traitement biologique, un dispositif (9, 10) d'alimentation du réacteur en oxygène, un dispositif (2, 4) d'alimentation du réacteur en eau résiduaire à surveiller, un dispositif (12) de mesure de la teneur en oxygène dissous dans le réacteur et un dispositif d'alarme relié au dispositif de mesure pour déclencher une alarme de toxicité en cas de diminution de la respiration, caractérisé par: un dispositif (3, 5) pour alimenter en permanence le réacteur par des boues activées en provenance de la station; et un dispositif de régulation (13) qui comporte un circuit comparateur (15) destiné à comparer la valeur mesurée de la teneur en oxygène dissous à une valeur minimale et à une valeur maximale prédéterminées, un circuit (37, 38, 40) de commande d'une pompe (4) d'alimentation du réacteur (1) en eau résiduaire, et un circuit de sélection (21–26) relié au circuit comparateur (15) et destiné à appliquer au circuit de commande un signal différent selon que la teneur en oxygène mesurée est inférieure à ladite valeur minimale, supérieure à ladite valeur maximale ou comprise entre les deux, de façon à diminuer ou augmenter le débit d'alimentation du réacteur en eau résiduaire lorsque la teneur mesurée devient, respectivement, inférieure à la valeur minimale ou supérieure à la valeur maximale.

6. Appareil selon la revendication 5, caractérisé en ce que ledit signal est formé par des impulsions de période fixe et de durée variable.

7. Appareil selon l'une quelconque des revendications 5 et 6, caractérisé en ce qu'il comporte un circuit de temporisation réglable (91 à 97) qui est destiné à commander au moins un circuit d'alarme (100, 103), qui est déclenché lorsque le débit d'alimentation du réacteur (1) en eau résiduaire atteint un maximum, par suite d'une augmentation de la teneur mesurée en oxygène dissous au-delà de la valeur maximale prédéterminée et qui est ramené à zéro lorsque la teneur mesurée en oxygène dissous redevient inférieure à ladite valeur maximale avant la fin de la temporisation.

8. Appareil selon l'une quelconque des revendications 5 à 7, caractérisé en ce que l'alimentation du réacteur (1) en eau résiduaire et en boues activées est assurée par des boucles d'alimentation rapides (2, 3).

## Claims

1. Process for detecting toxic substances in the waste water supplied to a biological treatment plant, process according to which the waste water to be analyzed is brought in contact with activated sludges taken from the biological treatment plant inside a reactor, oxygen being constantly supplied to the reactor and the quantity of oxygen dissolved in the reactor is measured in order to release a toxicity alarm signal in case of abnormal reduction of the respiration, characterized in that: the reactor is permanently supplied with said activated sludges, the measured quantity of oxygen dissolved in the reactor is permanently compared to preset maximum and minimum values; the flow of waste water supplied to the reactor is automatically regulated by being reduced or increased whenever the measured content value is respectively below the preset minimum value or above the preset maximum value, in order to keep substantially constant the load applied to the reactor; and the toxicity alarm is released whenever the measured content exceeds the maximum value while the waste water supply flow to the reactor is at a maximum.

2. Process according to claim 1, characterized in that the toxicity alarm is released whenever the measured dissolved oxygen content remains above said selected maximum value for a selected time period commencing when the waste water supply flow to the reactor reaches its maximum.

3. Process according to any one of claims 1 and 2, characterized in that the waste water supply flow to the reactor may be varied by predetermined thresholds between a minimum and a maximum.

4. Process according to any one of claims 1 and 2, characterized in that the waste water supply flow to the reactor may be varied continuously.

5. Apparatus for detecting toxic substances in the waste water supplied to a biological treatment plant, comprising a reactor (1) designed to contain activated sludges taken from the biological treatment plant, a device (9, 10) for supplying the reactor with oxygen, a device (2, 4) for supplying the reactor with waste water to be analyzed, a device (12) for measuring the quantity of oxygen dissolved in the reactor and an alarm device connected to the measuring device to release a toxicity alarm in case of reduction of the respiration, characterized by: a device (3,5) for permanently supplying the reactor with activated sludges taken from the plant; and a regulating device (13) comprising a comparator circuit (15) designed to compare the measured value of the dissolved oxygen content to preset minimum and maximum values, a control circuit (37, 38, 40) for controlling a pump (4) supplying waste water to the reactor (1), and a selector circuit (21, 26) connected to the comparator circuit (15) and designed to apply a different signal to the control circuit depending on whether the measured oxygen content is less than said minimum value, or more than said maximum value or between the two, in order to reduce or increase the waste water supply flow to the reactor when the measured content becomes respectively less than the minimum value or greater than the maximum value.

6. Apparatus according to claim 5, characterized in that said signal is formed by pulses of fixed period and variable duration.

7. Apparatus according to any one of claims 5 and 6, characterized in that it comprises an adjustable timing circuit (91 to 97) designed to control at least one alarm circuit (100, 103) which is activated when the waste water supply flow to the reactor (1) reaches a maximum as a result of an increase in the measured dissolved oxygen content above the selected maximum value, the timing circuit being resetted when said measured dissolved oxygen content returns to under said maximum value before said selected time period elapses.

8. Apparatus according to any one of claims 5 to 7, characterized in that the waste water and activated sludges supply to the reactor (1) is ensured by fast supply loops (2,3).

**Patentansprüche**

1. Verfahren zur Feststellung giftiger Substanzen im Abwasser, welches einer biologischen Behandlungsstation zugeführt wird, wonach das zu überwachende Abwasser in einem Reaktor mit aktiviertem Schlamm aus der biologischen Behandlungsanlage in Kontakt gebracht wird, wobei eine konstante Sauerstoffzufuhr zum Reaktor aufrecht erhalten wird und der Sauerstoffgehalt im Reaktor gemessen wird, um im Falle einer anormalen Verringerung der Atmung einen Giftigkeitsalarm auszulösen, dadurch gekennzeichnet, dass der Reaktor permanent mit den genannten aktivierten Schlämmen versorgt wird, dass der gemessene Wert des im Reaktor gelösten Sauerstoffes permanent mit einem minimalen und einem maximalen vorbestimmten Wert verglichen wird; dass die dem Reaktor zugeführte Menge des Abwassers automatisch reguliert wird und dabei erhöht oder erniedrigt wird, wenn der gemessene Gehalt den minimalen Wert unterschreitet oder den maximalen Wert überschreitet, sodass im wesentlichen eine konstante Charge im Reaktor aufrecht erhalten wird; und dass der Giftigkeitsalarm ausgelöst wird, wenn der gemessene Gehalt über dem genannten maximalen Wert bleibt, obwohl die dem Reaktor zugeführte Menge an Abwasser ein Maximum ist.

2. Verfahren nach Anspruch 1, dadurch gekennzeichnet, dass der Giftigkeitsalarm ausgelöst wird, wenn der Gehalt an gelöstem Sauerstoff über eine vorbestimmte Zeit, die ausgehend vom Moment, indem die dem Reaktor zugeführte Menge an Abwasser ihr Maximum erreicht, gezählt wird, grösser bleibt.

3. Verfahren nach einem der Ansprüche 1 und 2, dadurch gekennzeichnet, dass die dem Reaktor zugeführte Menge an Abwasser zwischen einem Minimum und einem Maximum um vorbestimmte Schritte variabel ist.

4. Verfahren nach einem der Ansprüche 1 und 2, dadurch gekennzeichnet, dass die dem Reaktor zugeführte Menge an Abwasser kontinuierlich veränderbar ist.

5. Vorrichtung zur Feststellung giftiger Substanzen im Abwasser, das einer biologischen Bearbeitungsstation zugeführt wird, mit einem Reaktor (1), der dazu bestimmt ist, aktivierte Bohrschlämme von der biologischen Behandlungsstation aufzunehmen, einer Sauerstoffzufuhrvorrichtung (9, 10) zum Reaktor, einer Vorrichtung (2, 4) zur Zuführung des zu überwachenden Abwassers zum Reaktor einer Messvorrichtung (12) zur Messung des im Reaktor gelösten Sauerstoffgehaltes und einer Alarmanlage, die mit der Messvorrichtung verbunden ist, um den Giftigkeitsalarm im Falle einer Herabsetzung der Atmung auszulösen, gekennzeichnet durch: eine Vorrichtung (3, 5) um den Reaktor permanent aktivierte Bohrschlämme aus der Station zuzuführen; und einer Reguliervorrichtung (13), die einen Vergleichskreis (15) aufweist, der dazu bestimmt ist, den gemessenen Wert des gelösten Sauerstoffes mit einem vorbestimmten minimalen und einem vorbestimmten maximalen Wert zu vergleichen, einer Kommandoschaltung (37, 38, 40) für eine Pumpe (4), die dem Reaktor (1) Abwasser zuführt und einer Wählschaltung (21 bis 26), die mit der Vergleichsschaltung (15) verbunden ist und dazu bestimmt ist, dem Kommandokreis unterschiedliche Signale zu übermitteln, je nachdem, ob der gemessene Sauerstoffgehalt unter dem genannten minimalen Wert, über dem genannte maximalen Wert oder zwischen diesen beiden Werten liegt, um die Menge des zugeführten Abwassers zu erniedrigen oder zu erhöhen, je nachdem, ob der gemessene Gehalt kleiner als der minimale oder grösser als der maximale Wert ist.

6. Vorrichtung nach Anspruch 5, dadurch gekennzeichnet, dass das Signal aus Impulsen fester Periode und unterschiedlicher Länge besteht.

7. Vorrichtung nach einem der Ansprüche 5 und 6, dadurch gekennzeichnet, dass sie einen regelbaren Zeitkreis (91 bis 97) aufweist, der dazu bestimmt ist, zumindest einen Alarmkreis (100, 103) zu betätigen, der ausgelöst wird, wenn die Menge des dem Reaktor (1) zugeführten Abwassers in der Folge eines Anstieges des gemessenen gelösten Sauerstoffgehaltes über den vorbestimmten maximalen Wert ein Maximum erreicht und der auf Null zurückgesetzt wird, wenn der gemessene Gehalt des gelösten Sauerstoffes vor dem Ende des Zeitablaufes unter diesen maximalen Wert wieder absinkt.

8. Vorrichtung nach einem der Ansprüche 5 bis 7, dadurch gekennzeichnet, dass die Versorgung des Reaktors (1) mit dem Abwasser und den aktivierten Schlämmen durch Schnellschlammzufuhrmittel gesichert ist.

Fig. 1

Fig. 2

0 114 780

0 114 780

Fig. 3

Fig. 4

Fig. 5

Fig. 6

13